(19) Europäisches Patentamt
European Patent Office
Office européen des brevets

(11) EP 0 726 895 B1

# (12) FASCICULE DE BREVET EUROPEEN

(45) Date de publication et mention de la délivrance du brevet: **05.07.2000 Bulletin 2000/27**

(21) Numéro de dépôt: **95900185.0**

(22) Date de dépôt: **03.11.1994**

(51) Int. Cl.⁷: **C07D 233/64**, C12P 17/10, A61K 31/415, A61K 7/42

(86) Numéro de dépôt international: **PCT/FR94/01272**

(87) Numéro de publication internationale: **WO 95/12581 (11.05.1995 Gazette 1995/20)**

## (54) PRODUIT PSEUDODIPEPTIDE POSSEDANT UN GROUPEMENT IMIDAZOLE ET APPLICATIONS

PSEUDODIPEPTID-PRODUKTE, DIE EINE IMIDAZOL-GRUPPE ENTHALTEN UND IHRE ANWENDUNGEN

PSEUDODIPEPTIDE PRODUCT HAVING AN IMIDAZOLE GROUPING AND APPLICATIONS

(84) Etats contractants désignés:
**BE CH DE ES GB GR IE IT LI MC NL PT**

(30) Priorité: **05.11.1993 FR 9313480**

(43) Date de publication de la demande: **21.08.1996 Bulletin 1996/34**

(73) Titulaires:
- **EXSYMOL S.A.M.**
  **MC-98000 Monte Carlo (MC)**
- **BABIZHAYEV, Marc**
  **74, Moscou, 127434 (RU)**

(72) Inventeurs:
- **BABIZHAYEV, Marc**
  **Moscow, 127434 (RU)**
- **SEGUIN, Marie-Christine**
  **F-06360 Eze (FR)**

(74) Mandataire: **Bonneau, Gérard**
**Cabinet Bonneau,**
**Conseil en Propriété Industrielle,**
**7, Avenue Gazan**
**06600 Antibes (FR)**

(56) Documents cités:
**EP-A- 0 143 746** **EP-A- 0 144 290**
**EP-A- 0 372 818**

- **JOURNAL OF ORGANIC CHEMISTRY., vol.56, no.19, 1991, EASTON US pages 5606 - 5610 J. F. MODDER ET AL 'Synthesis and X-ray structure of the chiral, polydentate cation binder N-(N-((5-methyl-2thienyl)met hylidene)-L-methionyl)histamine'**
- **ZEITSCHRIFT FüR CHEMIE, vol.9, no.4, 1969 page 144 H. AROLD ET AL 'Synthese einige Aminoacy-histamine und -tryptamine'**
- **CHEMICAL ABSTRACTS, vol. 90, no. 15, 9 Avril 1979, Columbus, Ohio, US; abstract no. 115121, M. L. PECK ET AL 'Radioprotective potential and chelating properties of glycylhistamine, an analog of histamine terminal peptides found in bee vendom' page 52 ; & TOXICON, vol.16, no.6, 1978 pages 690 - 694**
- **JOURNAL OF THE CHEMICAL SOCIETY, PERKIN TRANSACTIONS 2, no.1, Janvier 1994, LETCHWORTH GB pages 157 - 164 T. GADJA ET AL 'Multinuclear NMR and potentiometric study on tautomerism during protonation and Zn (II) complex formation of some imidazole-containing peptide derivatives'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.7 (HRS)/3.6

## Description

La présente invention concerne les produits pseudodipeptides, et en particulier un nouveau produit pseudodipeptide possédant un groupement imidazole, ainsi que les applications d'un tel produit dans les domaines thérapeutique, cosmétologie ou agro-alimentaire.

L'objet de l'invention est un nouveau produit pseudodipeptide à base d'imidazole, ledit produit étant choisi dans le groupe consistant en glutamyl-histamine, N-méthyl-2-amino-L-butyryl-histamine, 2-aminoisobutyryl-histamine, L-norleucylhistamine, L-2 aminooctyl-histamine, Boc-2-amino-L-pentyl-histamine, L-ornithyl-histamine, L-2,6-diaminopimélyl-histamine, N-acétyl-2-aminobutyryl-histamine, N-phénacétyl-2-aminobutyryl-histamine, L-arginyl histamine, L-prolyl-histamine, 4-hydroxy-L-prolyl-histamine, L-pyroglutamyl-histamine.

Le produit pseudodipeptide selon l'invention peut être considéré comme un produit de condensation entre un alpha aminoacide et l'histamine.

A ce titre, il peut donc être obtenu par différents procédés de synthèse aussi bien chimiques qu'enzymatiques.

Un procédé de préparation chimique adéquat pour la préparation du produit pseudodipeptide de l'invention, désigné de façon symbolique par AA-Hist (AA est l'acide aminé et Hist désigne l'histamine) se présente selon le schéma suivant:

$$AA + X + Y \rightarrow X\text{-}AA\text{-}Y \qquad (1)$$

$$X\text{-}AA\text{-}Y + Hist\ (.2Hcl) \rightarrow X\text{-}AA\text{-}Hist \qquad (2)$$

La première étape du procédé consiste à rendre l'acide aminé (AA) N-protégé par un groupement X, et O-activé par un groupement Y.

La N-protection est effectuée de préférence par le remplacement d'un atome d'hydrogène dans l'amine de l'acide aminé qui peut être un radical acyle ou acyloxy... Parmi les groupements protecteurs les plus intéressants on peut citer le benzyloxycarbonyle, (Z) le 9-fluorenyl-méthyloxycarbonyle (Fmoc), les radicaux benzyle, phthaloyle, 2-nitrophénylsulfényle et le trifluoroacétyle.

Bien qu'il soit possible de s'en dispenser, l'activation O est réalisée de préférence par estérification de la fonction carboxylique de l'acide aminé par un composé choisi dans le groupe consistant en: alcool de cyanométhyle, o-nitrophénol, 2,4,5-trichiorophénol, p-nitrophénol, 2,4-dinitrophénol, pentachlorophénol, pentafluorophénol, N-hydroxyphtalimide, N-hydroxysuccinimide, 1-hydroxypipéridine et 5-chloro-8-hydroxy-quinoline.

Parmi les modes d'O-activation on peut encore citer le procédé consistant à transformer l'acide carboxylique an chlorure d'acide, un hydrazide et un anhydride "mixte" ou symétrique.

Un mode d'activation particulier consiste à faire réagir du phosgène sur l'aminoacide AA conduisant à la formation du N-carboxy anhydride de cet aminoacide.

La deuxième étape du procédé de préparation est le couplage avec l'histamine qui peut se faire avec ou sans agent de couplage, en faisant réagir l'acide aminé N-protégé et O-activé (ou non) avec l'histamine de préférence sous forme de dichlorhydrate. Il faut noter que l'agent de couplage n'est pas indispensable avec un acide aminé O-activé.

Le couplage sans agent de couplage s'effectue dans un solvant organique (ce peut être par exemple du chloroforme,1,2-diméthoxy-ethane, du diméthylformamide...) en présence d'acide (par exemple acide acétique...) ou de base (par exemple triéthylamine...) dans un solvant hydroorganique (par exemple eau-pyridine ou eau-1,2-diméthoxyethane...) en présence de base (par exemple soude ou bicarbonate de sodium...) puis d'acide (par exemple acide chlorhydrique...); dans des conditions catalytiques (par exemple imidazole, N-ethylmorpholine...)...

Si un agent de couplage est utilisé, cet agent peut-être par exemple le dicyclohexyl-carbodiimide, le N-hydroxybenzotriazole et ses dérivés tel que le benzotriazolyloxy (trisdiméthyl-amino) phosphonium hexafluorophosphate (BOP), le 1-isobutyloxycarbonyl-2-isobutyloxy-1,2-dihydroquinoline, le carbonyldiimidazole, Woodward Reagent K, a-chlorovinyl ethyl ether, a,a-dichlorodiethyl Ether, dichloromethyl methyl ether, DCC et additifs, DCC-pentachlorophénol, DCC-pentafluorophénol, cyanamide, cetenimines et cétènes, sels d'oxazolinium, EEDQ ('1-ethoxycarbonyl-2-ethoxy-1,2-dihydroquinoléine), ynamines acylphosphoniums, triphénylphosphite et imidazole, complexes de cuivre II, SiC14.

Lorsque cela s'avère nécessaire, le groupement X ou N-protecteur est éliminé lors d'une troisième étape:

$$X\text{-}AA\text{-}Hist \rightarrow AA\text{-}Hist \qquad (3)$$

Cette élimination se fait, selon les groupements protecteurs, par hydrogénolyse, par réduction par le sodium dans l'ammoniac liquide, par hydrazinolyse, par acidolyse, par hydrolyse ou par voie enzymatique. La solution préférée

consiste à effectuer cette étape de déprotection par acidolyse par l'acide trifluoroacétique. Dans ce dernier cas, le produit pseudodipeptide est obtenu sous forme base après traitement par l'ammoniaque. En outre, lorsque cela est recherché, le composé sous sa forme base peut être mis en présence d'un sel de métal de transition pour former un chélate.

A titre d'exemple, le procédé de préparation de la L-Glutamyl-histamine peut se faire de la façon suivante :

A une suspension de 2,0 g (6,59 mmol) de Boc-Glu(OtBu)-OH contenant 1,456 g (7,91 mmol) de pentafluorophénol en solution dans 8 ml d'acétate d'éthyle A 0°C est ajoutée goutte à goutte 4 ml d'une solution de 1,631 g (7,91 mmol) de dicyclohexylcarbodiimide-urée dans l'acétate d'éthyle. L'agitation à 0°C est maintenue 30 mn puis 1 heure à température ambiante.

Le mélange réactionnel est filtré pour éliminer la dicyclohexyl-urée. La dicyclohexyl-urée est lavée à l'acétate d'éthyle et le filtrat est évaporé. Le résidu huileux est séché à la pompe à palettes pendant 2 heures.

3,1 g de Boc-Glu(OtBu)-OPfp sont obtenus sous forme d'un solide blanc.

A une suspension de 1,213 g (6,59 mmol) de chlorhydrate d'histamine dans 15 ml de diméthylformamide contenant 1,331 g (13,18 mmol) de N-Methyl-Morpholine à 0°C est ajoutée goutte à goutte une solution de Boc-Glu(OtBu)-OPfp dans 5 ml de diméthylformamide. Le mélange réactionnel est agité 2 heures à 0°C puis 1h30 à température ambiante. Le mélange réactionnel est filtré et le précipité lavé au diméthylformamide.

Le mélange est filtré pour éliminer le précipité de chlorhydrate de N-Méthyl-Morpholine et le précipité est lavé à l'acétate d'éthyle, le diméthylformamide est évaporé sous vide de la pompe à palettes à t≤40°C. 25 ml d'acétate d'éthyle sont ajoutés au résidu puis la fraction insoluble est éliminée par filtration.

Le produit est lavé avec 20 ml d'hydrogénocarbonate de sodium 0,5M, 10 ml d'hydrogénocarbonate de sodium 0,5M, 20 ml d'eau et 20 ml d'une solution saturée en chlorure de sodium.

Après séchage de la phase organique sur sulfate de sodium pendant 1 heure, l'évaporation conduit à l'obtention d'une poudre blanche.

3,234 g (Rdt>100%) de produit sont récupérés mais la CCM montre qu'il reste du phénol et de l'histamine qui seront éliminés lors de l'étape suivante.

A 1,12 g de Boc-Glu(OtBu)-Hist solide est ajouté 25 ml d'acide trifluoroacétique. L'agitation est maintenue 1 heure à température ambiante. Le solvant est évaporé à la pompe à palettes à t≤40°C.

12 ml d'éther éthylique sont ajoutés au produit qui est agité pendant 30 minutes à température ambiante. Il se forme un produit blanc à l'empâtage. L'éther est décanté , le résidu lavé avec 10 ml d'éther éthylique puis séché à la pompe à palettes.

Le résidu est ensuite dissous dans 7 ml d'éthanol puis de l'ammoniaque à 5% est ajoutée jusqu'à pH 7,5. 25 ml d'acétate d'éthyle sont alors ajoutés et le tout est placé à 0°C pour quelques heures.

Il se forme 2 phases qui sont séparées par décantation et la phase inférieure huileuse jaunâtre est évaporée. Il se forme une poudre blanche très hygroscopique. Le produit est recristallisé par éthanol/acétate d'éthyle. Le produit est séparé par filtration puis lavé par le mélange éthanol/acétate d'éthyle (1/1).

510 mg de produit sont obtenus (Rdt=65%).

Les produits peptoïdes peuvent être avantageusement préparés, en terme de rendement et de coût, en utilisant une méthode de synthèse tout ou en partie enzymatique.

Selon ce procédé, on peut proposer les schémas de synthèse suivants :

La réaction de couplage peut être décrite par l'équation suivante :

$$X\text{-}AA\text{-}Y + hist + enzyme \rightarrow X\text{-}AA\text{-}hist + Y$$

où X-AA-Y est un aminoacide N-protégé dont la préparation a déjà été décrite et où le groupement protecteur X peut être un radical acyle, acyloxy,...

Dans le cas présent, le groupement X permet aussi d'accroître la solubilité de l'AA dans le milieu réactionnel. Ceci peut diriger le choix de X de façon à augmenter la solubilité de l'AA dans les milieux organiques tels que :

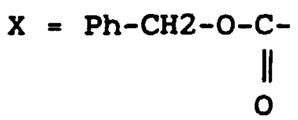
radical benzyloxycarbonyle

X-AA-Y est O-activé par estérification de la fonction carboxylique par un alcool choisi dans le groupe consistant en : alcools aliphatiques, et préférablement l'éthanol, halogénoalkylalcools tel que le 2,2,2-Trichloroéthanol, alcools aromatiques tel que le phénol, ainsi que tous les alcools cités précédemment pour l'activation en synthèse chimique. Cette liste exclue toutefois tous les alcools tertiaires.

La réaction de couplage avec l'histamine base (ou ses dérivés méthylés) ou un sel d'histamine (ou un de ses dérivés méthylés), par exemple le dichlorhydrate d'histamine, est réalisée dans une variété de solvants organiques, tels que les solvants hydrocarbonés aliphatiques (cyclohexane, heptane,..) ou aromatiques (toluène), les alcools tertiaires (t-Butanol, tert-Amylalcool), les halogénures d'alkyle (dichlorométhane), les éthers (isopropyléther), l'acétonitrile, le diméthyformamide ou le diméthylsulfoxide.
Ces solvants peuvent être utilisés seuls ou en association, anhydres ou en présence d'une faible quantité d'eau.

La réaction peut être conduite en présence ou non d'une base, telle que la triéthylamine.

Le catalyseur enzymatique est une hydrolase (lipase, protéase) d'origine microbienne ou animale ou végétale. Il peut être utilisé sous une forme pure ou non purifiée. On peut citer des qualités commerciales très peu coûteuses telles que des lipases, extraites de micro-organismes: Pseudomonas sp., Candida rugosa, Mucor, ou d'origine animale: lipase pancréatique de porc (LPP), des protéases: trypsine, chymotrypsine, substilisine, papaine,...

Ce catalyseur, non soluble dans le milieu réactionnel, est dispersé dans le solvant seul ou immobilisé sur un support inerte, afin de faciliter son recyclage.

- La réaction est effectué à une température comprise entre 4°C et 70°C, mais préférablement entre 35°C et 45°C sous agitation.
- Le produit de couplage est recueilli par filtration ou après extraction avec un solvant approprié.
- La déprotection et la purification peuvent être alors réalisés selon les procédés décrits pour la synthèse chimique.

Toutefois, l'étape de déprotection peut être réalisée par une réaction enzymatique selon la réaction :

$$\text{X-AA-hist + enzyme R- OH} \rightarrow \text{AA-hist + X-OR}$$

où R = H ou un radical alkyle.

Le protocole est similaire à celui décrit pour l'étape de couplage, toutefois, on voit que dans le cas particulier où R = H, la réaction est conduite dans l'eau.

Pour l'étape de couplage, il peut être envisagé des situations moins favorables, mais permettant de réduire les coûts de production de façon importante, en utilisant :

- un aminoacide non N-protégé (où X = H), O-activé comme précédemment
- un aminoacide N-protégé mais non O-activé (où Y = H)
- ou même l'aminoacide de départ (X = Y = H)

Les conditions opératoires sont similaires à celles décrites précédemment pour le dérivé N-protégé, O-activé.

Les caractéristiques structurales du produit pseudodipeptide de l'invention permettent d'obtenir un principe actif insensible à la désactivation enzymatique.

La caractéristique structurale essentielle est l'absence d'un groupement carboxyle sur les carbones en alpha de l'azote participant à la liaison peptidique,

D'une manière générale, la résistance à la désactivation enzymatique est favorisée par la présence de substituants stériquement encombrants sur le carbone terminal.

La résistance à la désactivation enzymatique est également renforcée lorsque l'amine terminale est incorporée dans une structure cyclique.

D'une manière plus générale encore, la résistance aux enzymes, et en particulier aux protéases, est renforcée lorsque l'alpha aminoacide du produit pseudodipeptide présente une structure qui diffère de celle des acides aminés naturels (on parle alors d'acides aminés non protéinogéniques ou non naturels). Ceci peut être réalisé par un choix judicieux de la stéréochimie du carbone terminal. En effet la présence d'un carbone terminal asymétrique permet d'obtenir une configuration absolue différente de celle des acides aminés naturels.

## Propriétés pharmacologiques

Les propriétés pharmacologiques du produit pseudodipeptide selon l'invention découlent de cette insensibilité à la désactivation enzymatique.

Ils possèdent avant tout des propriétés antioxydantes. Ils sont en effet capables de s'opposer au stress oxydatif, c'est à dire de s'opposer aux dommages provoqués par les espèces radicalaires sur les structures biologiques. Ces produits sont capables d'agir à plusieurs niveaux: ils peuvent réagir directement sur divers types de radicaux libres (R.L.), mais aussi sur des sous-produits toxiques du stress oxydatif. Il est également démontré que ces produits actifs peuvent également exercer une activité réparatrice vis à vis des altérations provoquées par les R.L. sur les structures

biologiques.

La réactivité d'un membre de cette famille de composés vis à vis de telle ou telle espèce radicalaire dépend d'éléments structuraux précis, sans qu'il soit possible à l'heure actuelle d'établir de règles de structure-activité. Il apparaît notamment qu'une faible différence structurale conduit à des modifications importantes du potentiel redox du pseudodipeptide, propriété physico-chimique directement liée au pouvoir antioxydant. Cette particularité rend compte de la diversité des propriétés observées pour cette famille de composés. A titre d'exemple, les résultats d'expérimentations in vitro obtenus avec deux pseudodipeptides sont présentés:

1°) Mise en évidence d'un pouvoir inhibiteur sur le radical libre OH°:
Protocole décrit par J.M.C. Gutteridge dans Biochemistry Journal, vol 224 (1984), p. 761-767:

- substrat d'oxydation: désoxyribose,
- système de production de radical hydroxyle OH°: EDTA/Fer, $H_2O_2$,
- détection: acide thiobarbiturique/malondialdéhyde (MDA)
- L'antioxydant de référence qui a été utilisé est la L-carnosine ou β-alanyl-l-histidine, en particulier pour le piégeage des espèces radiculaires comme OH-(radical hydroxyle) mais également $O_2$ (anion superoxyde). On peut trouver confirmation de ces propriétés dans les publications suivantes :
- « Free Radical Scavenging Activity of Carnosine » Free Radical Research Communications, Vol 14, N°4 (1991), pp 263-270.
- « Carnosine, homocarnosine and ansécrine : could they act as antioxidants in vivo ? » dans Biochemistry Journal, vol 264, (1989) pp 863-869.

| | % inhibition |
|---|---|
| Témoin (absence d'antioxydant) | 0 |
| β-alanyl-L-histidine (10mM) | 38 |
| L-prolyl-histamine (10mM) | 62 |
| L-glutamyl-histamine (10mM) | 0 |

2°) Mise en évidence d'un pouvoir inhibiteur sur l'anion superoxyde $O_2$°-:

- système de production d'anion superoxyde: xanthine oxydase/hypoxanthine (absence de fer)
- l'anion superoxyde est une substance réductrice. Elle est en particulier capable de réduire un substrat, le cytochrome c. Sa réduction est suivie par spectrophotométrie ultraviolette à 550 nm.

Le pourcentage d'inhibition est défini comme suit :

$$\frac{\text{vitesse de réduction du cytochrome c sans inhibiteur-vitesse de réduction avec inhibiteur}}{\text{vitesse de réduction du cytochrome c sans inhibiteur}} \times 100$$

| L-glutamyl-histamine (mM) | % d'inhibition |
|---|---|
| 1 | 3,5 |
| 5 | 14 |
| 10 | 24 |
| 20 | 35 |

Dans le système enzymatique générateur d'anion supéroxyde, la L-glutamyl-histamine est capable d'inhiber fortement la réduction du cytochrome c. Cette inhibition varie en fonction de la concentration. La L-prolyl-histamine, ou

l'anti-oxydant de référence, la β-alanyl-histamine, ne possèdent pas de pouvoir inhibiteur vis-à-vis de l'anion superoxyde.

Dans certains cas, lorsque l'aminoacide contient un groupement amine secondaire, le substituant de l'amine secondaire s'est avéré capable de renforcer certaines propriétés antioxydantes du produit pseudodipeptide, c'est le cas notamment lorsque le substituant est du tert-butoxycarbonle.

Une autre propriété importante de ces produits est leur capacité à protéger contre le stress oxydatif aussi bien des structures biologiques lipophiles (membranes cellulaires) ou hydrophiles (biopolymères tels que protéines, ADN),

Grâce à la présence du noyau imidazole et d'une amine nucléophile, certains des éléments de cette famille peuvent présenter une activité anti-glycation et donc s'opposer à la condensation non-enzymatique des sucres sur les protéines et ainsi empêcher leur dégradation.

La présence d'un élément de structure proche de celle de l'histamine permet, pour certains éléments de cette famille, d'obtenir un comportement inhibiteur des effets biologiques de l'histamine.

A l'inverse, dans certains cas très particuliers, il est possible d'obtenir un composé possédant une partie des propriétés biologiques de l'histamine.

Enfin, ces produits présentent également des propriétés cytostimulatrices et favorisent, de façon modérée, la multiplication de certains types de cellules. Cette propriété peut expliquer notamment le comportement immunostimulateur ou immunomodulateur de certains membres de cette famille de composés.

Selon ce mode d'action, les produits pseudodipeptides selon l'invention favoriseraient la cicatrisation en agissant sur les cellules du système immunitaire impliquées à un stade précoce dans la cicatrisation (lymphocytes, mastocytes, monocytes) et dont le rôle principal est la sécrétion des facteurs de croissance.

Ce pouvoir "immunostimulateur" est mis an évidence à l'aide d'un test in vitro sur splénocytes murins. Le protocole est extrait de : J. Kunert-Radek, H. Stepien, K. Lyson et M. Pawlikowski - "Effects of calcium channel modulators on the proliferation of mouse spleen lymphocytes in vitro" - Agents and Actions, vol.29, Nos 3-4 (1990), p. 254-258.

La prolifération cellulaire est suivie par la mesure du taux d'incorporation de thymidine tritiée dans les cellules, exprimé en nombre de désintégrations par mn, déduites du bruit de fond.

Les résultats obtenus avec un immunostimulant de référence, la concanavaline A, sont donnés à titre de comparaison.

L'effet immunostimulateur est exprimé par un Index de Stimulation (IS).

$$IS = \frac{\text{Nb de désintégrations par mn d'une suspension cellulaire additionnée de composé mitogène}}{\text{Nb de désintégrations par mn d'une suspension cellulaire de référence sans mitogène}}$$

Les valeurs correspondent à la moyenne de trois mesures.

On observe un effet immunostimulateur maximal pour une concentration de 5 µg/ml en L-glutamyl-histamine. En conclusion, on observe un effet immunostimulateur (prolifération cellulaire) modéré pour ce pseudodipeptide pour des concentrations comprises entre 5 et 10 µg/ml.

| Concentration en immunomodulateur (µg/ml) | | | | |
|---|---|---|---|---|
| | 2,5 | 5 | 10 | 25 |
| IS(L-glutamyl-histamine) | 7,8 | 42,7 | 42,1 | 5,6 |
| IS (concanavaline A) | 8,3 | 33,4 | 61,5 | 4,3 |

Cette activité est comparable à celle d'un mitogène de référence, la concanavaline A. Toutefois la préparation cellulaire utilisée pour ce test est hétérogène et contient en réalité plusieurs types de cellules mononucléées .

Afin de préciser le mode d'action des pseudodipeptides, une deuxième expérimentation a été réalisée sur une population homogène de monocytes humains.

La prolifération cellulaire a été évaluée de la même façon que précédemment:

| | Concentration en immunomodulateur (µg/ml) | | | |
|---|---|---|---|---|
| | 2,5 | 5 | 10 | 25 |
| IS(L-glutamyl-histamine) | 8,23 | 39,8 | 23,5 | 2,5 |
| IS (concanavaline A) | 4,82 | 29,6 | 21,1 | |

On met ainsi en évidence une stimulation optimum des monocytes pour la même concentration que précédemment. On observe également que ce pseudodipeptide est légèrement plus actif que la concanavaline A, d'un facteur de 1,34 en moyenne.

Ces résultats étayent l'hypothèse d'un mode d'action indirect sur les lymphocytes via l'activation des monocytes.

Dans la plupart des cas, la conservation in vivo de l'activité des produits pseudodipeptides de l'invention est liée à la préservation de l'intégrité de la molécule au contact des systèmes enzymatiques hydrolytiques, et en particulier des peptidases.

Toutefois, pour des applications particulières, une certaine sensibilité des produits pseudodipeptides de l'invention aux systèmes enzymatiques a été recherchée. C'est le cas où l'amino-acide contient une amine secondaire substituée par un des radicaux suivants

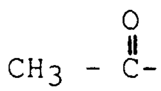

ou

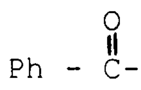

Le produit actif obtenu par exemple dans le premier cas (N-acétyl-2-aminobutyryl-histamine), est susceptible d'être hydrolysé de façon enzymatique in vivo pour reformer un nouveau composé pseudodipeptide toujours actif :

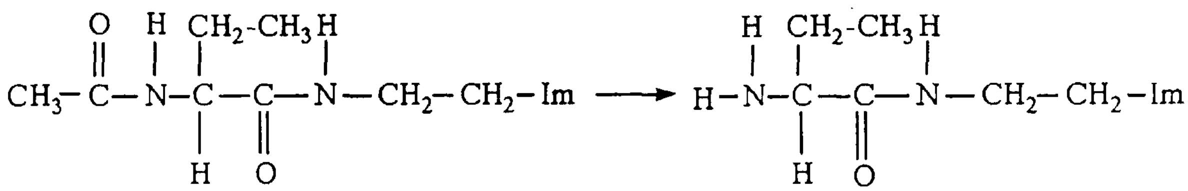

On n'obtient alors qu'une protection temporaire du composé peptoïde, mais c'est aussi une façon de reformer "in-situ" la fonction amine primaire du peptoïde et ainsi de restaurer certaines propriétés liées à la présence d'une amine nucléophile, ionisable, à pH physiologique, dans la molécule. On peut citer l'effet anti-glycation associé, en partie, à la capacité d'un composé aminé à se lier à l'aide d'une liaison covalente sur un sucre réducteur.

Cette stratégie peut être utile lorsqu'il est souhaitable : de modifier la polarité du peptoïde de départ (afin de le rendre compatible avec une formulation particulière par exemple), d'éviter la présence d'un groupement ionisable à pH physiologique sur la molécule, ou enfin de réduire la réactivité du peptoïde vis-à-vis d'autres espèces chimiques (incompatibilité avec la présence d'une espèce électrophile), présentes dans une formulation.

Cette stratégie permet aussi d'améliorer, dans certains cas, la biodisponibilité et la pharmacocinétique de cette catégorie de produits actifs, résultant en une potentialisation de l'effet pharmacologique.

**Applications thérapeutiques et cosmétologiques**

L'ensemble des propriétés énoncées ci-dessus conduit à des applications thérapeutiques et cosmétologiques appréciables des produits pseudodipeptides selon l'invention.

Les propriétés antioxydantes des pseudodipeptides selon l'invention permettent de proposer ces produits pour le traitement des pathologies associées au "stress oxydatif".

Parmi celles-ci, une application thérapeutique importante est le traitement de la cataracte. Les causes des différentes cataractes sont diverses. Les mécanismes impliqués dans ces pathologies, qu'elles soient du type "cataracte sénile" ou "cataracte du diabétique" sont regroupés en deux catégories: les mécanismes oxydatifs (M. A. Babizhayev, A.I. Deyev, L.F. Lindberg, "Lipid peroxidation as a possible cause of cataract", Mechanisms of Ageing Dev., Vol 44 (1988); P. 69-89), et les mécanismes de réticulation du type glycation (T.J. Lyons, G. Silvestri, J.A. Dunn, D.G.Dyer, J.W. Baynes "Role of glycation of lens crystallins in diabetic and non-diabetic senile cataracts", Diabetes Vol. 40, N° 8, (1991), P. 1010-1015).

Comme on l'a vu précédemment, les propriétés antioxydantes des pseudodipeptides s'exerçant en particulier par leur activité anti-radicaux libres et type "péroxydase" et également par leur activité anti-glycation, font des produits pseudodipeptides selon l'invention des produits efficaces pour soigner la cataracte. La capacité de ces composés à exercer une activité réparatrice vis à vis des altérations provoquées par les R.L. sur les structures biologiques est particulièrement importante dans le traitement de la cataracte car elle se traduit par une régression des opacités du cristallin.

Les produits pseudodipeptides selon l'invention peuvent s'opposer aux phénomènes oxydatifs responsables de l'athérosclérose. Dans cette pathologie, l'oxydation de particules lipoprotéiques de faible densité (LDL) circulant dans le sang est responsable de la fragmentation de la partie protéique (apoprotéine B) comme de la fraction lipidique de ces particules. Les fragments formés induiraient l'apparition de formes cellulaires anormales (monocytes et macrophages chargés en cholestérol) capables de s'agréger sur les parois des vaisseaux sanguins et de former la plaque d'athérome.

Les produits pseudodipeptides selon l'invention seraient de plus, particulièrement adaptés au traitement de cette maladie dans la mesure où il a été mis récemment en évidence que des phénomènes de glycation sont également impliqués dans la genèse de la plaque d'athérome (Ref. T.J. Lyons - "Glycation and oxidation - A role in the pathogenesis of Atherosclerosis" - American Journal of Cardiology, vol.71, N° 6 (1993), p. 1326-1331).

Les produits pseudodipeptides selon l'invention peuvent également s'opposer au processus de cancérogenèse dans la mesure où il a été démontré que les espèces radicalaires dérivées de l'oxygène sont responsables de la coupure ou de la modification des brins d'ADN, ces transformations pouvant être A l'origine de l'évolution des cellules saines en cellules cancéreuses.

De même, les propriétés antioxydantes des produits pseudodipeptides selon l'invention permettent d'indiquer ces produits pour le traitement des états inflammatoires pathologiques, et en particulier pour le traitement de l'arthrite rhumatisante. En effet, la détérioration du liquide synovial est un symptôme caractéristique de l'arthrite de type inflammatoire et on a pu montrer que la dégradation d'un de ses constituants essentiels, l'acide hyaluronique, était dû à un "stress oxydatif". Des études plus récentes (Ref. B. Halliwell and J.M.C. Gutteridge - "Chronic inflammation and the auto immune deseases" - Free Radicals in Biology and Medecine - B. Halliwell and J.M.C. Gutteridge Eds - Clarenton Press (1989), Oxford, p. 422-438) ont également mis en cause des phénomènes de péroxydation lipidique mis en jeu dans ce processus et qui expliqueraient l'action bénéfique des produits selon l'invention.

Les propriétés antioxydantes des produits pseudodipeptides selon l'invention peuvent également être exploitées en traitement annexe d'une radiothérapie. Cet effet radioprotecteur, déjà connu pour la β-alanyl-histidine repose également sur les propriétés cytostimulantes de ce type de composé, notamment vis-à-vis des cellules de la moelle osseuse, très sensibles aux rayonnements utilisés en radiothérapie.

Selon des données récentes, certains symptômes épileptiques pourraient provenir de lésions provoquées par les radicaux libres oxygénés sur certaines régions du cerveau (Ref. G.R. Jackson, K. Werrbach-Perer, J.R. Perez-Polo - "Role of nerve growth factor in oxidant-antioxidant balance and neuronal injury - II - A conditioning lesion paradigon" - Journal of Neuroscience Research, vol.25, N°3 (1990), p.369-374). Le pouvoir régénérant des tissus (nerveux en l'occurrence) des produits de l'invention est un élément important dans cette pathologie associée à une dégénérescence du tissu nerveux. Ces produits pourraient également être indiqués pour le traitement de la maladie de Parkinson dans laquelle serait impliqué un "stress oxydatif" touchant le tissu cérébral.

Certaines affections vasculaires et notamment l'endotoxémie, sont associées à la surproduction d'une espèce radicalaire: le radical nitrite oxyde NO°. L'action de ce radical, produit par les cellules endothéliales et les cellules des muscles lisses des vaisseaux sanguins, se traduit par une vasodilatation chronique. Cet état peut notamment être très préjudiciable lors de traitements impliquant des agents vasoconstricteurs. Dans les cellules, une enzyme, la NO-synthase, catalyse la transformation d'un acide aminé, la L-arginine, en NO°. Un pseudodipeptide antioxydant selon l'invention incorporant le radical L-arginyl (ou un dérivé $N^G$-méthyl-L-arginyl) dans sa structure, serait capable d'agir directement sur NO°, mais également sur l'enzyme responsable de sa synthèse, par un comportement d'inhibiteur de la catalyse enzymatique.

Au niveau de la peau, les propriétés antioxydantes des produits pseudodipeptides de l'invention peuvent être exploitées pour neutraliser les effets des espèces radicalaires oxygénées générées par le rayonnement lumineux. Ils s'opposeront ainsi efficacement aux réactions photoallergiques (les espèces radicalaires induisent au niveau de la peau la formation de molécules allergisantes). En association avec un principe actif sensible à l'oxydation (ex: Chlorpromazine), ils préviendront sa transformation en un composé toxique.

Ce principe trouve se meilleure application lors des traitements par photochimiothérapie de certaines maladies de la peau. En effet ces traitements reposent sur l'utilisation d'un photosensibilisant (ex: psoralène) qui, sous l'effet d'un rayonnement, exerce une action bénéfique (interaction avec l'ADN), mais qui, malheureusement, s'accompagne de la formation d'espèces radicalaires, responsables d'effets secondaires indésirables.

Les produits de l'invention peuvent également être indiqués pour s'opposer à l'apparition de symptômes cutanés chez des individus souffrant de porphyrie, car les porphyrines potentialisent les dommages occasionnés par les R.L.

Ils pourront également s'opposer à la formation de lésions cutanées liée au "stress oxydatif" chez les personnes souffrant de maladies auto-immunes tel que le Lupus Erythémateux Chronique (SLE).

Ils s'opposeront également efficacement aux effets du "coup de soleil": érythèmes, oedèmes et formation de cellules caractéristiques au niveau de la peau. Les propriétés antioxydantes des composés selon l'invention peuvent bien sûr être utilisées pour la prévention du vieillissement cutané. En effet, de multiples arguments expérimentaux, analytiques et épidémiologiques, militent en faveur de la théorie selon laquelle l'accumulation des dommages biochimiques provoqués par les R.L. constitueraient le processus essentiel du vieillissement. Il est notamment clair que l'exposition au rayonnement solaire, responsable de la formation d'espèces radicalaires dérivées de l'oxygène, est une cause du vieillissement cutané prématuré.

Enfin, il a été démontré expérimentalement que ces composés s'opposaient à d'autres phénomènes caractéristiques du tissu cutané vieillissant :

- La réticulation non-enzymatique de protéines telles que le collagène ou l'élastine par les sucres (V.M. Monnier - "Nonenzymatic glycosylation, the Maillard reaction and the aging process" - Journal of Gerontology, vol. 45, N°4 (1990), p. B105-111),
- La formation de complexes lipoprotéiques: lipofushines (réticulation par des sous-produits du stress oxydatif)

Il a été démontré que la L-arginine était capable de s'opposer à la réticulation non-enzymatique (cet amino acide peut se condenser sur les sucres mais aussi sur certains sous-produits dicarbonylés du stress oxydatif). Un pseudodipeptide selon l'invention incorporant dans sa structure un radical L-arginyle sera donc particulièrement indiqué pour s'opposer à ces mécanismes du vieillissement tissulaire.

Une autre série d'applications relève des propriétés cytostimulantes des produits pseudodipeptides selon l'invention et, comme il l'a été démontré, de leurs propriétés immunostimulatrices. Ces propriétés permettent de favoriser la régénération tissulaire et la cicatrisation, et d'une manière générale, de réguler les fonctions faisant intervenir les effecteurs de la réponse immunitaire.

Ils peuvent ainsi être utilisés pour favoriser la régénération des tissus conjonctifs cutanés perturbés. Ils favorisent la réparation des muqueuses après brûlures ou après traitements chimio et radiothérapiques.

Selon ce principe, ces produits peuvent également être indiqués pour la prévention et le traitement des rides.

Les propriétés cytostimulatrices et régénératrices des peptoïdes s'exercent tout particulièrement vis à vis des tissus musculaires où l'on rencontre des concentrations élevées d'un dipeptide naturel apparenté, la β-alanyl-histidine. Bien que le rôle physiologique de ce composé n'est pas, à l'heure actuelle, parfaitement établi, il est étroitement lié au métabolisme musculaire. Ainsi, les produits de l'invention pourraient participer à l'amélioration de la contractilité des muscles, réguler la contraction cardiaque. On peut également utiliser ce type de propriétés pour le traitement de certaines dégénérescences musculaires telles que la myodystrophie de Dushen.

Les propriétés cicatrisantes des produits pseudodipeptides selon l'invention trouvent leur application dans de nombreux domaines. On peut citer le traitement des ulcères gastriques, pour lequel un produit dipeptide apparenté à la carnosine, a donné de bons résultats (M. Ito, T. Tanaka and Y. Suzuki - "Effect of N-(3-aminoproprionyl)-L-histidinato zinc (Z-103) on healing and hydrocortisone-induced release of acetic acid ulcers in rats with limited food-intake-time" - Japaneese Journal of Pharmacology, vol.54 (1990), p.513-521). En outre, les propriétés antioxydantes et anti-inflammatoires des produits de l'invention sont utiles pour le traitement de cette pathologie. Un chélate de zinc selon l'invention, tel qu'il a été décrit précédemment, est particulièrement indiqué pour cette application.

Les propriétés cicatrisantes des produits pseudodipeptides selon l'invention sont également particulièrement indiquées pour le traitement des atteintes cornéennes. Ils pourront être administrés en traitement postopératoire, après incision de la cornée pour la correction de la myopie, par exemple.

Ils peuvent également être avantageusement utilisés pour le traitement des pathologies de "l'oeil sec" en favorisant la cicatrisation de l'épithélium cornéen mais également grâce à un comportement de "larme artificielle": protection des tissus lésés contre le "stress oxydatif" (état, inflammation, rayonnement U.V.), incorporation dans des formulations favorisant la restauration du film lacrymal.

Des compositions contenant les produits pesudodipeptides de l'invention pourront aussi, de par leur action

immunostimulante et régénératrice des tissus, s'opposer aux phénomènes de dégénérescence rétinienne. Ils verront leurs effets renforcés par le fait qu'une composante "stress oxydatif" intervient dans cette pathologie (Ref. R.E. Anderson, L.M. Rapp, R.D. Wiegand - Current Eye Research, vol.3 (1984), p.223-227).

Les propriétés immunostimulatrices des produits pseudodipeptides de l'invention peuvent enfin être mises à profit pour la potentialisation des vaccins en remplacement des adjuvants classiquement utilisés pour renforcer la réponse immune chez l'homme (sels d'aluminium, extraits d'origine bactérienne, monophosphoryl A...) dont les effets secondaires ne sont pas négligeables (RK Gupta, EH Relyveld, EB Lindblad, B Bizzini, S Ben-Efraim and CK Gupta "Adjuvants - a balance between toxicity and adjuvancity" Vaccine, vol.11, n°3 (1993), P.293-306).

Comme il a déjà été mentionné, certains des pseudodipeptides répondant à la formule générale donnée plus haut, peuvent se comporter comme des inhibiteurs de l'histamine. Parmi les applications thérapeutiques exploitant cette propriété, on peut citer :

- Un antagoniste de l'histamine pourra s'opposer à l'agrégation plaquettaire avec des applications pour le traitement des affections du système vasculaire périphérique. En effet, il a été prouvé que l'histamine est un médiateur intracellulaire favorisant l'agrégation plaquettaire (S.P. Saxena, L.J. Brandes, A.B. BeckerK.J. Simons, F.S. La Bella and J.M. Gerrard, Science, vol. 243, n°24 (1989), P. 1596-1599). L'histamine est également un médiateur intracellulaire impliqué dans la croissance et la multiplication cellulaire (JR. Chanda, A.K. Ganguly Cancer Letters, vol.34 (1987), P. 207). Un pseudodipeptide antagoniste selon l'invention pourra donc être utilisé pour réguler la multiplication cellulaire, notamment au niveau des tissus cicatriciels hypertrophiques (chéloïdes).
- L'histamine est aussi un messager extra-cellulaire intervenant dans de nombreux processus biologiques. Ainsi un antagoniste selon l'invention pourra être appliqué pour le traitement des allergies, de l'inflammation, de certains dysfonctionnements cardiaques,... et pour toutes les pathologies où la libération excessive d'histamine est mise en cause.

Il pourrait ainsi être particulièrement bénéfique d'inclure les produits pseudodipeptides selon l'invention dans les parfums et déodorants afin de s'opposer aux réactions allergiques, et en particulier aux chocs anaphylactiques, provoqués par certaines compositions fortement odorantes.

Certains éléments de cette famille de composés peuvent, à l'inverse du cas précédent, posséder certaines des propriétés de l'histamine (comportement "pro-histaminique"). Un comportement d'antagoniste des récepteurs H2 de l'histamine permet d'inhiber l'activation des neutrophiles et par conséquent la production excessive de radicaux libres par ces cellules (R. Burde, R Seifert, A.Buschaeur, G. Schultz, "Histamine inhibits activation of human neutrophils and HL-60 leukemic cells via H2-receptors" Naunyn-Schmiedebergs Arch. of Pharmacology , vol.340, n°6 (1989), P 671-678) . Ceci permet d'envisager l'utilisation de tel pseudodipeptides pour le traitement de certains états inflammatoires pathologiques.

L'histamine favorise également la multiplication et la croissance cellulaire (G. Kahlson, E. Rosengren, C. Steinhardt Journal of Physiology, vol. 151 (1960), P. 131). Un composé "pro-histaminique" pourrait ainsi favoriser la régénération cellulaire.

Une série d'applications repose sur la capacité de certains des produits pseudodipeptides selon l'invention possédant dans leur structure un radical L-prolyle ( et dérivés) à s'opposer à une synthèse anormale de collagène et à son accumulation dans les tissus. Cette propriété particulière pourrait être liée à une analogie structurelle avec un dipeptide naturel, la L-glycyl-L-proline favorisant la dégradation du collagène, mais aussi à leurs propriétés antioxydantes car le stress oxydatif à récemment été mis en cause dans la surproduction de collagène (J.C. Geesin, L.J. Hendricks, P.A. Falkenstein, J.S. Gordon, R.A. Berg "Regulation of collagen synthesis by ascorbic acid: characterization of the role of ascorbate-stimulated lipid peroxidation" Archives of Biochemistry and Biophysics, vol. 290, n°1 (1991) P. 127-132).

L'accumulation de collagène dans les tissus cardiaques est notamment une des complications majeures du diabète.

Ce type de composés est indiqué pour réduire les chéloïdes associés à une surproduction de collagène lors du processus de cicatrisation. Dans la mesure où les pseudodipeptides selon l'invention peuvent également se comporter comme des anti-histaminiques et qu'il a récemment été démontré que l'histamine stimule la production de collagène (A. Hatamochi, H. Ueki, C. Mauch, T. Krieg "Effect of histamine on collagen and collagen m-RNA production in human skin fibroplast", Journal of Dermatologic Sciences, Vol 2 (1991), P. 407-412), les peptoïdes selon l'invention sont particulièrement adaptés à cette application.

**<u>Applications comme stabilisant et conservateur</u>**

L'excellente tolérance vis-à-vis des pseudodipeptides selon l'invention, et leurs propriétés antioxydantes et cytostimulantes modérées permettent de proposer ces produits pour la conservation et la protection de substances

sensibles à l'oxydation, de matières alimentaires ou d'organes et de tissus conservés ex vivo.

On peut citer la prévention de l'oxydation des liposomes afin d'améliorer leur stabilité et d'éviter la formation de sous-produits toxiques, la protection de l'acide hyaluronique incorporé dans des formules cosmétiques contre l'action dépolymérisante des radicaux libres, la protection des huiles et des produits alimentaires oxydables, de produits diététiques.

Les produits de l'invention permettent d'améliorer la conservation des vaccins du sang et du serum, la préservation d'organes voués à la transplantation (avec une référence particulière pour le coeur).

**Revendications**

1. Produit pseudodipeptide à base d'imidazole, ledit produit étant choisi dans le groupe consistant en glutamyl-histamine,N-méthyl-2-amino-L-butyryl-histamine, 2-aminoisobutyryl-histamine, L-norleucyl-histamine, L-2-aminooctyl-histamine, Boc-2-amino-L-pentyl-histamine, L-ornithyl-histamine, L-2,6-diaminopimélyl-histamine, N-acétyl-2-aminobutyryl-histamine, N-phénacétyl-2-aminobutyryl-histamine, L-arginyl histamine, L-prolyl-histamine, 4-hydroxy-L-prolyl-histamine, L-pyroglutamyl-histamine.

2. Procédé chimique de préparation du produit selon la revendication 1 comprenant les étapes suivantes:

    - on rend un alpha aminoacide N-protégé par un groupement X,
    - on rend ledit alpha aminoacide O-activé par un groupement Y,
    - on couple ledit alpha aminoacide N-protégé et O-activé avec de l'histamine, et
    - on élimine X ou non selon le produit pseudodipeptide recherché.

3. Procédé selon la revendication 2 dans lequel l'alpha aminoacide est rendu O-activé par estérification de la fonction carboxylique dudit aminoacide.

4. Procédé selon la revendication 3 dans lequel l'étape d'estérification pour rendre l'alpha aminoacide O-activé est réalisée par la réaction avec un composé choisi dans le groupe consistant en: alcool de cyanométhyle, o-nitrophénol, 2,4,5-trichlorophénol, p-nitrophénol, 2,4-dinitrophénol, pentachlorophénol, pentafluorophénol, N-hydroxyphtalimide, N-hydroxysuccinimide, 1-hydroxypipéridine et 5-chloro-8-hydroxy-quinoline.

5. Procédé enzymatique de préparation du produit selon la revendication 1 consistant à coupler un alpha aminoacide avec de l'histamine en présence d'un catalyseur enzymatique du type hydrolase.

6. Procédé selon la revendication 5 dans lequel ledit catalyseur enzymatique est une hydrolase choisie dans le groupe consistant en lipases extraites de micro-organismes ou d'origine animale, et en protéases.

7. Application du produit selon la revendication 1 pour obtenir un médicament destiné à soigner la cataracte.

8. Application du produit selon la revendication 1 pour obtenir un médicament destiné à soigner l'athérosclérose.

9. Application du produit selon la revendication 1 pour obtenir un médicament destiné à soigner les états inflammatoires.

10. Application du produit selon la revendication 1 pour obtenir un médicament destiné à soigner les maladies de la peau associées au stress oxydatif telles que la photoallergie, la porphyrie, et le Lupus Erythémateux.

11. Application du produit selon la revendication 1 pour obtenir un médicament contre l'endotoxémie et autres maladies vasculaires associées à une surproduction du radical libre NO.

12. Application du produit selon la revendication 1 pour obtenir un médicament destiné à la cicatrisation des tissus tels que les tissus cutanés, la muqueuse gastrique et les tissus oculaires.

13. Application du produit selon la revendication 1 pour obtenir un médicament pour le traitement des lésions gastriques dans lequel ledit produit pseudodipeptide est sous forme de chélate avec un atome de zinc.

14. Application du produit selon la revendication 1 pour obtenir un médicament agissant comme agent radioprotecteur.

15. Application du produit selon la revendication 1 pour obtenir un médicament utilisé comme inhibiteur de la synthèse du collagène.

16. Médicament résultant de l'application selon la revendication 15 pour le traitement de certaines complications du diabète, et pour la réduction des chéloïdes lors du processus de cicatrisation de la peau.

17. Application du produit selon la revendication 1 pour obtenir une composition thérapeutique ou cosmétique destinée à la régénération et au rajeunissement des tissus tels que les muqueuses et les tissus cutanés.

18. Application du produit selon la revendication 1 pour obtenir un médicament agissant comme immunostimulant pour la potentialisation des vaccins.

19. Application du produit selon la revendication 1 pour obtenir un médicament capable de s'opposer à l'agrégation plaquettaire.

20. Application du produit selon la revendication 1 pour obtenir un médicament anti-allergique.

21. Application du produit selon la revendication 1 pour obtenir pour la conservation et la stabilisation de substances sensibles à l'oxydation, de matières alimentaires ou d'organes et de tissus conservés ex vivo.

**Claims**

1. A pseudodipeptide product based upon imidazole, said product being selected in the group consisting in glutamyl-histamine, N-méthyl-2-amino-L-butyryl-histamine, 2-aminoisobutyryl-histamine, L-norleucyl-histamine, L-2-aminooctyl-histamine, Boc-2-amino-L-pentyl-histamine, L-ornithyl-histamine, L-2,6-diaminopimélyl-histamine, N-acétyl-2-aminobutyryl-histamine, N-phénacétyl-2-aminobutyryl-histamine, L-arginyl histamine, L-prolyl-histamine, 4-hydroxy-L-prolyl-histamine, L-pyroglutamyl-histamine.

2. A chemical process for the preparation of the product according to claim 1, comprising the following steps :

- Making said alpha aminoacid N-protected by a group X,
- Making said alpha aminoacid O-activated by a group Y,
- Coupling said N-protected and O-activated alpha amino acid with histamine, and
- Either eliminating or not X according to the desired pseudodipeptide product.

3. Process according to claim 2, wherein the alpha aminoacid is made O-activated by an esterification of the carboxylic acid function of said aminoacid.

4. Process according to claim 3, wherein the esterification step of making the alphaaminoacid O-activated is carried out through the reaction with a compound selected in the group consisting in : cyanomethyl alcohol, o-nitrophenol, 2, 4, 5-trihlorophenol, p-nitrophenol, 2, 4-dinitrophenol, pentachlorophenol, pentafluorophenol, N-hydroxyphtalamide, N-hydroxysuccinimide, 1-hydroxypiperidine and 5-chloro-8-hydroxy-quinoline.

5. An enzymatic process for the preparation of the product according to claim 1, consisting in coupling of an alpha aminoacid with histamine in presence of an enzymatic catalyst of the hydrolase type.

6. Process according to claim 5, wherein said enzymatic catalyst is an hydrolase chosen in the group consisting in lipases extracted from micro-organisms or from animal origin, and in proteases.

7. Application of the product according to claim 1, in order to obtain a medicine intended for cataract treatment.

8. Application of the product according to claim 1, in order to obtain a medicine intended for the treatment of atherosclerosis.

9. Application of the product according to claim 1, in order to obtain a medicine intended for the treatment of inflammatory states.

10. Application of the product according to claim 1, in order to obtain a medicine intended for the treatment of skin

deseases associated to an oxidative stress such as photoallergy, porphyria, and Lupus Erythematosus.

**11.** Application of the product according to claim 1, in order to obtain a medicine against endotoxemia and other vascular deseases associated to an overproduction of the free radical NO.

**12.** Application of the product according to claim 1, in order to obtain a medicine intended for the healing of tissues such as the cutaneous tissues, the gastric mucosa and the ocular tissues.

**13.** Application of the product according to claim 1, in order to obtain a medicine for the treatment of the gastric lesions in which said pseudodipeptide product is under the form of a chelate with an atom of zinc.

**14.** Application of the product according to claim 1, in order to obtain a medicine acting as a radioprotective agent.

**15.** Application of the product according to claim 1, in order to obtain a medicine used as an inhibitor of the synthesis of collagen.

**16.** Application of the product according to claim 1, for the treatment of some diabetes complications, and for the reduction of the cheloids during the skin healing process.

**17.** Application of the product according to claim 1, in order to obtain a therapeutical or cosmetic composition for the regeneration and rejuvenation of the tissues such as mucosa and cutaneous tissues.

**18.** Application of the product according to claim 1, in order to obtain a medicine acting as an immunostimulator for the potentiation of vaccines.

**19.** Application of the product according to claim 1, in order to obtain a medicine able to counteract platelets aggregation.

**20.** Application of the product according to claim 1, in order to obtain a medicine against allergies.

**21.** Application of the product according to claim 1, in order to preserve and stabilize the substances sensitive to oxidation, of food products or organs and of tissues kept ex vivo.

**Patentansprüche**

**1.** Pseudodipeptid-Produkt auf der Grundlage von Imidazol, das ausgewählt ist aus der aus den folgenden Verbindungen bestehenden Gruppe: Glutamyl-histamin, N-Methyl-2-amino-L-butyryl-histamin, 2-Aminoisobutyryl-histamin, L-Norleucyl-histamin, L-2-Aminooctyl-histämin, Boc-2-amino-L-pentyl-histamin, L-Ornithyl-histamin, L-2,6-Diaminopimelyl-histamin, N-Acetyl-2-aminobutyryl-histamin, N-Phenacetyl-2-aminobutyryl-histamin, L-Arginyl-histamin, L-Prolyl-histamin, 4-Hydroxy-L-prolyl-histamin, L-Pyroglutamyl-histamin.

**2.** Chemisches Verfahren zur Herstellung des Produkt nach Anspruch 1, bestehend aus den folgenden Schritten:

- man macht eine alpha-Aminosäure durch eine X-Gruppe N-geschützt,
- man macht diese alpha-Aminosäure durch eine Y-Gruppe O-aktiviert,
- man koppelt diese N-geschützte und O-aktivierte alpha-Aminosäure mit Histamin und
- man entfernt X oder entfernt es nicht, und zwar je nach dem gewünschten Pseudodipeptid-Produkt.

**3.** Verfahren nach Anspruch 2, in dem die alpha-Aminosäure durch Veresterung der Carboxylgruppe dieser Aminosäure O-aktiviert gemacht wird.

**4.** Verfahren nach Anspruch 3, in dem der Schritt der Veresterung, mit dem die alpha-Aminosäure O-aktiviert gemacht werden soll, durch die Reaktion mit einer Verbindung durchgeführt wird, die aus der aus den folgenden Verbindungen bestehenden Gruppe ausgewählt ist: Cyanmethylalkohol, o-Nitrophenol, 2,4,5-Trichlorphenol, p-Nitrophenol, 2,4-Dinitrophenol, Pentachlorphenol, Pentafluorphenol, N-Hydroxyphthalimid, N-Hydroxysuccinimid, 1-Hydroxypiperidin und 5-Chlor-8-hydroxy-chinolin.

**5.** Enzymatisches Verfahren zur Herstellung des Produkts nach Anspruch 1, das darin besteht, daß eine alpha-Aminosäure mit Histamin in Gegenwart eines enzymatischen Katalysators vom Typ Hydrolase gekoppelt wird.

**6.** Verfahren nach Anspruch 5, in dem der enzymatische Katalysator eine Hydrolase ist, die aus der Gruppe ausgewählt ist, die aus aus Mikroorganismen extrahierten Lipasen oder Lipasen tierischen Ursprungs und aus Proteasen besteht.

**7.** Anwendung des Produkts nach Anspruch 1 für die Herstellung eines Arzneimittels für die Behandlung des grauen Stars.

**8.** Anwendung des Produkts nach Anspruch 1 für die Herstellung eines Arzneimittels für die Behandlung der Atherosklerose.

**9.** Anwendung des Produkts nach Anspruch 1 für die Herstellung eines Arzneimittels zur Behandlung entzündlicher Zustände.

**10.** Anwendung des Produkts nach Anspruch 1 für die Herstelllung eines Arzneimittels zur Behandlung von mit oxidativem Streß verbundenen Hautkrankheiten wie Photoallergie, Porphyrie und Lupus erythematodes.

**11.** Anwendung des Produkts nach Anspruch 1 für die Herstellung eines Arzneimittels gegen Endotoxämie und andere mit einer Überproduktion des freien Radikals NO verbundene Gefäßerkrankungen.

**12.** Anwendung des Produkts nach Anspruch 1 für die Herstellung eines Arzneimittels für die Vernarbung von Geweben wie Hautgeweben, Magenschleimhaut und Augengeweben.

**13.** Anwendung des Produkts nach Anspruch 1 für die Herstellung eines Arzneimittels zur Behandlung von Magenverletzungen, bei dem das Pseudodipeptid-Produkt in Chelat-Form mit einem Zinkatom vorliegt.

**14.** Anwendung des Produkts nach Anspruch 1 für die Herstellung eines Arzneimittels, das als Strahlenschutzmittel wirkt.

**15.** Anwendung des Produkts nach Anspruch 1 für die Herstellung eines Arzneimittels, das als Mittel zur Hemmung der Kollagensynthese verwendet wird.

**16.** Arzneimittel, das sich aus der Anwendung nach Anspruch 15 ergibt, für die Behandlung von manchen Komplikationen des Diabetes und für die Reduzierung der Cheloiden im Hautvernarbungsprozess.

**17.** Anwendung des Produkts nach Anspruch 1 für die Herstellung einer therapeutischen oder kosmetischen Zusammensetzung zur Regenerierung und Verjüngung von Geweben wie Schleimhäuten und Hautgeweben.

**18.** Anwendung des Produkts nach Anspruch 1 für die Herstellung eines Arzneimittels, das als Immunstimulanz für die Potentialisierung von Impfstoffen wirkt.

**19.** Anwendung des Produkts nach Anspruch 1 für die Herstellung eines Arzneimittels, das der Thrombozytenaggregation entgegenwirken kann.

**20.** Anwendung des Produkts nach Anspruch 1 für die Herstellung eines antiallergischen Arzneimittels.

**21.** Anwendung des Produkts nach Anspruch 1 für die Konservierung und Stabilisierung von hinsichtlich Oxidation empfindlichen Substanzen, Nahrungsmitteln oder ex vivo konservierten Organen und Geweben.